# EUROPEAN PATENT APPLICATION

(11) **EP 1 188 770 A1**
(43) Date of publication of application: **20.03.2002**
(21) Application number: 00937225.1
(22) Date of filing: 14.06.2000
(51) Int. Cl.: C07K 14/745, C07K 17/14

(54) **SUBSTANCE BINDING TO THE SUBSTRATE OF ACTIVATED BLOOD COAGULATION FACTOR IN COMPETITION WITH THIS FACTORTO THEREBY REGULATE THE R EACTION BETWEEN THE ACTIVATED BLOOD COAGULATION FACTOR AND THE SUBSTRATE, A PROCESS FOR PRODUCING THE SUBSTANCE AND BLOOD COAGULATION FACTOR-ADSORBENT WITH THE USE OF THE**

(30) Priority: 14.06.1999 JP 16745399; 07.03.2000 JP 2000062629
(71) Applicant: FUJIMORI KOGYO CO., LTD., Tokyo 103-0002 (JP); CHISSO CORPORATION, Osaka-shi, Osaka-fu 530-0005 (JP)
(72) Inventor: HOSOKAWA, Kazuya, Kawasaki-shi, Kanagawa 211-0002 (JP)
(74) Representative: Lee, Nicholas John
(86) International application number: JP0003863
(87) International publication number: WO0077048

(57) **Abstract**

This invention provides a substance capable of inhibiting only an aimed enzymatic activity and a blood coagulation factor adsorbent using this substance. To be specific, this invention is accomplished by a substance which is capable of depressing the reaction between an activated blood coagulating factor and a substrate thereof by binding itself to said substrate in competition with said activated blood coagulating factor and a blood coagulation factor adsorbent using this substance.

## Description

### Technical Field

This invention relates to a substance capable of inhibiting a reaction between an activated blood coagulating factor and a substrate thereof by binding itself to said substrate in competition with said activated blood coagulating factor, a method for the production of the substance, and a blood coagulating factor adsorbent using the substance.

### Background Art

In the endeavors toward curing and preventing the thrombosis such as disseminated intravascular clotting (DIC), a host of preventives against thrombogenesis such as antiplatelet drugs and anticoagulant drugs are being used.

As effective preventives against thrombogenesis among other anticoagulant drugs, such serine protease inhibitors as activated blood coagulation factor II, activated blood coagulation factor VII, activated blood coagulation factor IX, and activated blood coagulation factor X which inhibit serine protease activity have been heretofore used. The serine protease inhibitors are capable of inhibiting the activity of a serine protease by binding themselves to the active region of the serine protease.

The conventional activated blood coagulation factor inhibitors such as serine protease inhibitors, however, have had a difficulty in selectively inhibiting exclusively the enzymatic activity of a given activated blood coagulation factor because several species of activated blood coagulation factor possess a similar structure in the active region.

Further, the preventives against thrombogenesis mentioned above are varied in action mechanism. To date, a substance which closely resembles an activated blood coagulation factor in structure and which inhibits the reaction between the substrate of the activated blood coagulation factor and the activated blood coagulation factor by binding itself to said substrate in competition with said activated blood coagulation factor, thereby prevents intravascular thrombogenesis has never been developed.

### Disclosure of the Invention

The first object of this invention consists in providing a substance capable of inhibiting the reaction between an activated blood coagulation factor and a substrate thereof by binding itself to said substrate in competition with said activated blood coagulation factor and a method for the production of the substance.

The use of this substance permits substantial inhibition of the activity of an activated blood coagulation factor. The substance, therefore, is not only highly useful as a preventive for thrombogenesis of new mechanism but also effectively useful in the control of enzymatic activities in the fermentation industry and in other fields such as the process for purification of useful proteins.

The second object of this invention consists in a blood coagulation factor adsorbent which is formed by fixing as a ligand on a carrier a substance capable of inhibiting the reaction between an activated blood coagulation factor and a substrate thereof by binding itself to said substrate in competition with said activated blood coagulation factor.

To date, a blood coagulation factor adsorbent having the substance mentioned above fixed as a ligand on a carrier and a method for purifying a blood coagulation factor by the use of the adsorbent have never been developed.

The third object of this invention is to provide a method for purifying a blood coagulation factor without entailing any conspicuous possibility of contaminating fibrinogen and foreign proteins, etc. therein.

The present inventor, after pursuing a series of diligent studies concerning the problems of the prior art as mentioned above, has found that exclusively a target enzymatic activity can be selectively inhibited by using as an enzymatic activity depressant represented by a thrombogenesis preventive a substance- capable of inhibiting the reaction between an activated blood coagulation factor and a substrate thereof by binding itself to said substrate in competition with said activated blood coagulation factor and further discovered that when a given blood coagulation factor is refined by using a blood coagulation factor adsorbent formed by having the substance fixed as a ligand on a carrier, the possible contamination of fibrinogen and foreign proteins, etc. in the blood coagulation factor can be repressed conspicuously. This invention has been perfected based on this knowledge.

This invention is composed of the following items (1) - (11).
(1) A substance capable of inhibiting a reaction between an activated blood coagulating factor and a substrate thereof by binding itself to said substrate in competition with said activated blood coagulating factor.
(2) A substance set forth in the item (1) mentioned above, wherein said substance is formed from an amino acid sequence having one or more amino acids of an activated blood coagulation factor deleted therefrom, substituted therefor, or added thereto.
(3) A substance set forth in the item (1) or (2) mentioned above, wherein said substance is formed by anhydridizing the active serine residue site of the activated blood coagulation factor possessing an active serine residue.
(4) A substance set forth in the item (3) mentioned above, wherein said activated blood coagulation factor possessing an active serine residue is activated blood coagulation factor X.
(5) A substance set forth in the item (3) mentioned above, wherein said activated blood coagulation factor possessing an active serine residue is activated blood coagulation factor IX.
(6) A substance set forth in the item (3) mentioned above, wherein said activated blood coagulation factor possessing an active serine residue is activated blood coagulation factor VII.
(7) A method for the production of a substance set forth in any of the items (1) - (6) mentioned above, characterized by performing the following steps
   1. a step of causing an active serine residue site of an activated blood coagulation factor possessing an active serine residue to react with a synthetic inhibitor,
   2. a step of performing an alkali treatment at a pH value in the range of 11.0 - 13.5, and
   3. a step of performing a recovering operation, in the order mentioned above sequentially and allowing at least the step of performing collection to proceed with permitting coexistence of at least one compound selected from the group consisting of polyhydric alcohols and saccharides with a salt or an amphoteric electrolyte.
(8) A blood coagulation factor adsorbent having a substance set forth in any of the items (1) - (6) mentioned above fixed on a carrier.
(9) A blood coagulation factor adsorbent set forth in the item (8) mentioned above, wherein the carrier is in the form of particles.
(10) A blood coagulation factor adsorbent set forth in the item (8) or (9) mentioned above, wherein the carrier is cellulose.
(11) A method for purifying a blood coagulation factor, characterized by using a blood coagulation factor adsorbent set forth in any of the items (8) - (10) mentioned above.

### Best Mode of Embodying the Invention

Now, this invention will be described in detail below.

As the first aspect of this invention, the substance capable of inhibiting a reaction between an activated blood coagulating factor and a substrate thereof by binding itself to said substrate in competition with said activated blood coagulating factor is preferred to be such a substance as avoids exhibiting an enzymatic activity. Further, this substance is preferred to have a structure resembling the structure of a substance of the activated blood coagulation factor.

The substance capable of inhibiting a reaction between an activated blood coagulating factor and a substrate thereof by binding itself to said substrate in competition with said activated blood coagulating factor does not need to be particularly defined. As concrete examples of the substance satisfying this description, a substance formed from an amino acid sequence having one or more amino acids of an activated blood coagulation factor deleted therefrom, substituted therefor, or added thereto and a substance formed by anhydridizing the active serine residue site of an activated blood coagulation factor possessing an active serine residue may be cited.

As concrete examples of the substance which is formed from an amino acid sequence having one or more amino acids of an activated blood coagulation factor deleted therefrom, substituted therefor, or added thereto, a substance formed by substituting asparagine for aspartic acid in the active site of an activated blood coagulation factor and a substance formed by substituting alanine for the active serine in the active site of an activated blood coagulation factor may be cited.

In this invention, when, in the substance formed from an amino acid sequence having one or more amino acids of an activated blood coagulation factor deleted therefrom, substituted therefor, or added thereto, the activated blood coagulation factor is activated blood coagulation factor VII, activated blood coagulation factor IX, or activated blood coagulation factor X, the substance possesses a conspicuous effect of preventing thrombogenesis. For this invention, therefore, the activated blood coagulation factor is preferred to be activated blood coagulation factor VII, activated blood coagulation factor IX, or activated blood coagulation factor X.

The expression "substance having the active serine residue site of an activated blood coagulation factor possessing an active serine residue anhydridized" refers to a substance what has modified the active serine residue that is an active center of the activated blood coagulation factor to dehydroalanine.

This invention does not impose any particular restriction either on the activated blood coagulation factor possessing an active serine residue. When, in the substance having the active serine residue site of an activated blood coagulation factor possessing an active serine residue anhydridized, the activated blood coagulation factor possessing the active serine residue is activated blood coagulation factor VII, activated blood coagulation factor IX, or activated blood coagulation factor X, this substance possesses a prominent effect of preventing thrombogenesis. In this invention, therefore, the activated blood coagulation factor possessing an active serine residue is preferred to be activated blood coagulation factor VII, activated blood coagulation factor IX, or activated blood coagulation factor X.

An activated blood coagulation factor and an activated blood coagulation factor possessing an active serine residue which are raw materials herein do not need to be particularly limited the kind of method used for production thereof. Specifically, such raw materials as obtained by purifying from blood plasma and then activating them, or by the operation of gene recombination may be effectively used in this invention.

The method of this invention for producing the substance which has anhydridized an active serine residue site of an activated blood coagulation factor possessing an active serine residue, which is hereinafter referred to occasionally as "anhydridized coagulation factor", does not need to be particularly limited. As a concrete example of this method, a method which converts the active serine residue of an activated blood coagulation factor possessing the active serine residue by a chemical synthesis technique into dehydroalanine may be cited.

The present invention prefers this method to perform the following the 1st-3rd steps
a step of causing an active serine residue site of an activated blood coagulation factor possessing an active serine residue to react with a synthetic inhibitor (1st step),
a step of performing an alkali treatment at a pH value in the range of 11.0 - 13.5 (2nd step), and
a step of performing collection (3rd step) in the order mentioned above sequentially and allowing at least the step of performing collection to proceed with permitting coexistence of at least one compound selected from the group consisting of polyhydric alcohols and saccharides with a salt or an amphoteric electrolyte.

The method of production mentioned above will be described below by citing as an example thereof a case of using phenylmethanesulfonyl fluoride (PMSF) as a synthetic inhibitor. The method may be represented by the following reaction formula (1).

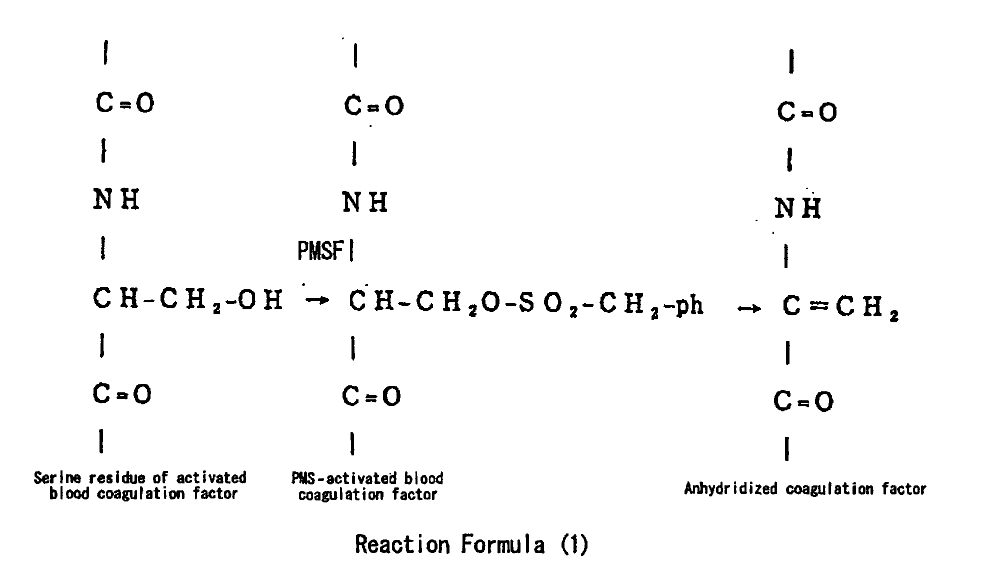

Now, the method for the production of an anhydridized coagulation factor of this invention will be described below along the course of 1st-3rd steps mentioned above.

Incidentally, this method of production contemplated by this invention all 1st-3rd steps to proceed with permitting coexistence of at least one compound selected from the group consisting of polyhydric alcohols and saccharides with a salt or an amphoteric electrolyte and prefers at least the step for performing collection to proceed with permitting coexistence of at least one compound selected from the group consisting of polyhydric alcohols and saccharides with a salt or an amphoteric electrolyte.

### (1) 1st step

The 1st step consist in forming an ester linkage between the active serine residue binding site of an activated blood coagulation factor and a synthetic inhibitor with a view to inactivating the activation of the activated blood coagulation factor possessing an active serine residue and comprises a step of causing the active serine residue binding site of the activated blood coagulation factor to react with the synthetic inhibitor and a step of purifying and separating the product of the reaction.

The synthetic inhibitor which can be used for the method of production of this invention does not need to be particularly limited but is only required to be capable of reacting with the active serine residue of the activated blood coagulation factor possessing the active serine residue and consequently forming an ester linkage as mentioned above. As concrete examples of the synthetic inhibitor, various sulfonyl fluorides such as PMSF, 2-phenylethane-1-sulfonylfluoride, methanesulfonylfluoride, and p-toluenesulfonyl (tosyl) fluoride, and tosylchloride, diisopropylfluoro-phosphoric acid (DFP), 3,4 - dichloroisocoumarin (3,4 - DCI), L -1-chloro-3-[4-tosylacid]-7-amino-2-heptanon-hydrochloric acid (TLCK), and L-1- chloro -3- [4-tosylacid] -4- phenyl -2-butanone (TPCK) may be cited.

For the purpose of causing the synthetic inhibitor mentioned above to react with an activated blood coagulation factor possessing an active serine residue, the synthetic inhibitor may be used as it is without modification. Otherwise, the synthetic inhibitor may be dissolved in advance of use in such a solvent as methanol, acetone, ethanol, propanol, isopropanol, butanol, propan-2-ol, dimethyl formaldehyde, or dimethyl sulfoxide.

When the synthetic inhibitor is added to excess, an operation of separating and removing the excess will be required later. The addition of the synthetic inhibitor, therefore, is properly carried out while the confirmation of the activity of the activated blood coagulation factor possessing the active serine residue is continued until the activity reaches preferably a level of not more than 3%, more preferably not more than 1%.

The reaction solvent to be used in the 1st step is preferably buffer containing sodium chloride or the buffer having further added thereto several kinds of ion such as potassium ion, calcium ion, and magnesium ion. Additionally, the buffer has a pH value preferably in the range of 2 - 10, more preferably in the range of 4 - 8.

As concrete examples of the buffer, phosphate buffer, carbonate buffer, bicarbonate buffer, tris buffer, citric acid-sodium phosphate buffer, succinic acid-sodium hydroxide buffer, phthalic acid-sodium hydroxide buffer, imidazole-hydrochloric acid buffer, borate buffer, physiological saline solution, and Good's buffer may be cited. The buffer system is so adjusted to have a pH value in the range of 2 - 10.

The reaction temperature in the 1st step does not need to be particularly limited but is only required to avoid affecting the stability of the activated blood coagulation factor possessing an active serine residue. In this invention, it is preferably in the range of -30 - 50°C, more preferably in the range of 4 - 40°C.

The product which is obtained by the step of causing the active serine residue binding site of the activated blood coagulation factor to react with synthetic inhibitors may be separated and purified by using a known method.

The method to be used for the purpose of separation and purification does not need to be particularly restricted. As concrete examples of the method, gel filtration, ion-exchange chromatography, affinity chromatography, ultrafiltration, and dialysis may be cited.

The separation and purification will be explained below by citing as an example a case of performing the purification of the product by gel filtration. By adding a reaction solution including the active serine residue binding site of an activated blood coagulation factor and a synthetic inhibitor to a column packed with gel particles, which can be sephadex, Biogel, or agarose gel, etc., swelled with the same solvent as used in the reaction and then continuing the flow of the solvent to the column. Then first, products that are high molecular solutes, secondly, products that are low molecular solutes are eluted, thereby inducing first the product of a high molecular solute and, after some delay, the emission of a synthetic inhibitor which is a low molecular solute, with the result that the product and the excess synthetic inhibitor will be separated from each other.

### (2) 2nd step

The 2nd step consists in dissociation the synthetic inhibitor from the product obtained at the 1st step and, at the same time, modifying a serine residue to dehydroalanine and using it as an anhydridized coagulation factor. In terms of operation, this step is operated by performing an alkali treatment on the product at a pH value in the range of 11.0 - 13.5.

At the 2nd step, an alkali liquid may be directly added to the product obtained at the 1st step so as to prepare the product solution having a pH value in the range of 11.0-13.5. Otherwise, an alkali liquid or an alkali substance may be added to the solution obtained by dissolving the product in a solvent in advance so as to prepare the product solution having a pH value in the range of 11.0 - 13.5.

As the solvent mentioned above, the buffer that is used as the reaction solvent at the 1st step mentioned above may be used.

The product solution prepared as described above is kept standing for a prescribed period at a temperature in the range of -30 - 50°C, preferably in the range of 4 - 40°C.

In the 2nd step of this invention, if the pH value falls short of 11.0, the shortage will profoundly reduce the velocity of the dissociation of the synthetic inhibitor from the product and the velocity of the conversion of the serine residue to the dehydroalanine.

If the temperature of the product solution at the 2nd step falls short of -30°C, the shortage will have the possibility of freezing the product solution itself. Conversely, if this temperature exceeds 50°C, the excess will possibly suffer the product and/or the substance dissociated the synthetic inhibitor from the product, i.e., anhydridized coagulation factor, to succumb to protein denaturation.

### (3) 3rd step

The 3rd step comprises a step of performing a regenerating treatment on the anhydridized coagulation factor obtained at the 2nd step coexistent at least one compound selected from the group consisting of polyhydric alcohols and saccharides and a salt or an amphoteric electrolyte and a step of separating and purifying the regenerated anhydridized coagulation factor.

By the regenerating treatment, the stereostructure of the anhydridized coagulation factor can be reformed to the same stereostructure as the original structure of the activated blood coagulation factor possessing an active serine residue.

The regenerating treatment does not need to be particularly restricted but may be performed by any of the known methods. A method which comprises adjusting the pH value of the product solution after completion of the 2nd step to a level in the range of 4 - 10 with the buffer used as the reaction solvent at the 1st step mentioned above and then keeping the solution to stand at a temperature in the range of -30-50°C for a prescribed period and a method which comprises adjusting the pH value at a level in the range of 4 - 10 by dialysis may be cited as concrete examples of the method mentioned above.

Then, the regenerated anhydridized coagulation factor is purified and separated by depriving the reaction system of the at least one compound selected from the group consisting of polyhydric alcohols and saccharides which has been made to coexist with the reaction system and further the salt or amphoteric electrolyte which needs to be expelled from the system and other impurities, when one kind selected from salt or amphoteric electrolyte such as NaCl or phosphate is allowed to be contained in the elute to be used in the operation of final extraction of the anhydridized coagulation factor, it possibly does not need to be separated and removed deliberately.

The method for separating for purification of the at least one compound selected from the group consisting of polyhydric alcohols and saccharides which has been made to coexist with the reaction system and further the salt or amphoteric electrolyte which must be expelled from the system does not need to be particularly restricted but may be selected from among the methods known heretofore. For example, dialysis, ultrafiltration, gel chromatography, ion-exchange chromatography, and affinity chromatography, are available for the separation.

A typical operation of dialysis consists in causing permeation of at least one compound selected from the group consisting of polyhydric alcohols and saccharides from the solution of a regenerated anhydridized coagulation factor through a membrane such as cellulose into a solvent having a pH value in the range of 4 - 10.

Then, the other method for separating for purification of impurities does not need to be particularly restricted but may be selected from among the methods known heretofore.

A method which comprises concentrating a solution containing a regenerated anhydridized coagulation factor by YM 10 membrane, for example, then passing to wash the concentrated solution through a benzamidine sephalos column etc., equilibrated with a solvent having a pH value in the range of 4 - 10 (the buffer used as the reaction solvent at the 1st step mentioned above may be used as the solvent), further eluting the adsorbate on the column with a benzamidine solution adjusted to a pH value in the range of 4 - 10 (the benzamidine solution mentioned above may contain such a salt as sodium chloride, potassium chloride, calcium chloride, or magnesium chloride for the purpose of effecting specific adsorption of a target protein), and then dialyzing the eluate with a solvent with a pH value in the range of 4 - 10 (the buffer used as the reaction solvent at the 1st step mentioned above may be used as the solvent) for the purpose of removing the benzamidine, thereby effecting extraction of the regenerated anhydridized coagulation factor and a method which comprises effecting separation by ultrafilitration and performing gel filtration by the use of a sephadex column may be cited as concrete examples of the method.

As concrete examples of the polyhydric alcohol (inclusive of sugar alcohols) required to coexist in the reaction system in all the 1st-3rd steps or at least in the 3rd step of performing an operation or collection, tetritols (such as, for example, erythritol, D-threitol, L-threitol, and D,L-threitol), pentitols (such as, for example, ribitol, D-arabinitol, L-arabinitol, D,L-arabinitol, and xylitol), hexytols (such as, for example, allitol, dulcitol (galactitol), sorbitol (D-glucitol), L-glucitol, D,L-glucitol, D-mannitol, L-mannitol, D,L-mannitol, D-altritol, L-altritol, D,L-altritol, D-iditol, and L-iditol), heptitol, maltitol, lactitol, glycerin, ethylene glycol, diethylene glycol, triethylene glycol, propylene glycol, dipropylene glycol, 1,3-butylene glycol, neopentyl glycol, pentamethylene glycol, hexamethylene glycol, pentaerythritol, dipentaerythritol, tripentaerythritol, trimethylol ethane, trimethylol propane, anhydrouse enneaheptitol, 1,4-butane diol, 1,2,4-butan triol, and 1,2,6-hexane triol may be cited.

As concrete examples of the saccharide which is required to coexist in the reaction system during all the first - 3rd steps or during at least the 3rd step of performing an operation of collection, glycerin aldehyde dioxyacetone, threose, erythrulose, erythrose, arabinose, ribulose, ribose, xylose, xylulose, lyxose, glucose, fructose, mannose, idose, sorbose, gulose, talose, tagalose, galactose, allose, psicose, altrose, and sucrose may be cited.

In this invention, at least one compound selected from the group consisting of the polyhydric alcohols and the saccharides mentioned above may be used.

Further, in this invention, at least one compound selected from the group consisting of glycerin, ethylene glycol, and sucrose is preferably used.

The ratio of the at least one compound selected from the group consisting of polyhydric alcohols and saccharides mentioned above to be used as determined under conditions of 23°C in air temperature and 50% in relative humidity is not less than 5%, preferably not less than 15%, based on the whole of the reaction solution in terms of volume ratio in the case of a liquid sample, or in terms of weight ratio in the case of a powdery, particulate, or solid sample.

Even when the ratio falls short of 5%, it is made possible to perform the second and the 3rd step infallibly and attain satisfactory manifestation of the effect aimed at by relatively heightening the concentration of the salt or amphoteric electrolyte to be used at the same time. The ratio (i.e. the concentration) of the at least one compound selected from the group consisting of polyhydric alcohols and saccharides mentioned above to the whole of the reaction solution is preferred to be properly decided at an appropriate level depending on the kind of compound so as to ensure full manifestation of the effect aimed at. For the sake of this decision, it is necessary to consider the kind and the concentration of a salt or an amphoteric electrolyte to be used at the same time.

The salt or the amphoteric electrolyte which is used in the method for production according to this invention is intended to promote anhydridization of an activated coagulation factor possessing an active serine residue without inducing coagulation association of protein in an alkali treatment performed in a high pH range and effect regeneration of the anhydridized coagulation factor without inducing coagulation · association during returning the pH value from the high pH range to around neutrality at the 3rd step, owing to the joint use thereof with the at least one compound selected from the group consisting of polyhydric alcohols and saccharides mentioned above. The amount thereof to be used, therefore, does not need to be particularly restricted but is only required to be capable of attaining such a salt concentration (ionic strength) and such a dielectric constant as befit the purpose mentioned above.

As concrete examples of the salt or the amphoteric electrolyte to be used in this invention, water-soluble salts and amphoteric electrolytes including halogenated alkali metals such as sodium chloride and potassium chloride, hydrogenated alkaline earth metals such as magnesium chloride and calcium chloride, salts of inorganic acids such as ammonium chloride, ammonium sulfate, sodium carbonate, potassium carbonate, magnesium carbonate, ammonium carbonate, calcium carbonate, sodium hydrogen carbonate, calcium hydrogen carbonate, potassium hydrogen carbonate, ammonium hydrogen carbonate, sodium phosphate, disodium hydrogen phosphate, potassium dihydrogen phosphate, diammonium hydrogen phosphate, sodium borate, and potassium borate, salts of organic acids such as sodium citrate, potassium citrate, magnesium citrate, calcium citrate, ammonium citrate, sodium phthalate, potassium phthalate, magnesium phthalate, calcium phthalate, ammonium phthalate, sodium succinate, potassium succinate, magnesium succinate, calcium succinate, ammonium succinate, sodium acetate, potassium acetate, calcium acetate, magnesium acetate, and ammonium acetate, and amino acids destined to form such amphoteric electrolytes as glycin and alanine may be cited.

In this invention, the salts and the amphoteric electrolytes enumerated above may be used either singly or in the form of a mixture of two or more members.

Among other salts and amphoteric electrolytes cited above, alkali metal salts, inorganic salts, and amphoteric electrolytes having low molecular weights can be used particularly advantageously in the method for production of this invention because they are readily soluble in water, easily adjustable to the ionic strength (salt concentration) and the dielectric constant that best fit the concentration of the coexisting at least one compound selected from the group consisting of polyhydric alcohols and saccharides mentioned above, and capable of facilitating (or simplifying) the process of purification and separation (such as, for example, dialysis).

The concentration in the reaction solution of the salt or the amphoteric electrolyte used in the method of production accordding to this invention is properly not less than 0.2M, preferably not less than 0.5M. Even when this concentration falls short of 0.2M, it is made possible to perform the second and the 3rd step and attain satisfactory effect aimed at by relatively heightening the concentration thereof in the same reason as the at least one compound selected from the group consisting of polyhydric alcohols and saccharides mentioned above.

The anhydridized coagulation factor contemplated by this invention may be synthesized by a method other than the method described above such as, for example, a method which consist in using guanidine hydrochloride (Gdn-HCl) during the course of reaction in the place of PMSF as a reaction inhibitor.

The second aspect of this invention concerns a blood coagulation factor adsorbent having fixed on a carrier as a ligand a substance which is capable of depressing the reaction between an activated blood coagulating factor and a substrate thereof by binding itself to said substrate that is the first aspect of invention in competition with said activated blood coagulating factor.

The blood coagulation factor which the blood coagulation factor adsorbent of this invention adsorbs is the substrate of an activated blood coagulation factor which is subjected to the relevant repression of reaction.

To explain this adsorption by citing an example, when the active serine residue site of an activated blood coagulation factor X is a blood coagulation factor adsorbent having an anhydridized substance fixed on a carrier, the substance subjected to the adsorption is a blood coagulation factor VIII and the blood coagulation factor adsorbent can be used for separating and purifying an activated blood coagulation factor X.

The carrier to be used in the blood coagulation factor absorbent of this invention may be any of the carriers known heretofore. As concrete examples of the carrier, cellulose, agarose, chitosan, and vinyl polymers may be cited. The carrier to be used in this invention does not need to be particularly limited in terms of shape. It is preferably in the form of particles and more preferably in the form of cellulose.

The method for fixing (through reaction) the carrier and the ligand, the concentration of the fixed ligand, and the kind of a spacer to be used are attained by adopting techniques suitably selected from a wide range of techniques known heretofore.

The third aspect of this invention concerns a method for purifying a blood coagulation factor by the use of a blood coagulation factor adsorbent that forms the second aspect of this invention.

The purifying contemplated by this invention may be effected by any method so long as the method essentially makes use of the blood coagulation factor. For example, by packing a column with the blood coagulation factor adsorbent and passing a liquid containing a target blood coagulation factor through the column, it is made possible to separate the target blood coagulation factor and other components.

The conditions for the separation may be properly selected so as to suit the kind of blood coagulation factor and the solid state properties and behaviors of the liquid containing the blood coagulation factor.

### Example

Now, this invention will be described more specifically below with reference to working examples thereof.

### Example 1

### 1) Production of anhydridized activated blood coagulation factor X

To a solution of 10.5 mg of an activated blood coagulation factor X derived from human blood plasma in 10 ml of 5 mM phosphate buffer/0.1M NaCl/pH 6.5, 30 µl of 7% solution of phenyl methyl sulfonyl fluoride (PMSF) in methanol was added at intervals of 30 minutes until the total activity fell below 0.1%.

The produced solution was cooled to 0°C and the cooled solution and 0.5 ml of 1M NaOH added thereto were left reacting with each other for 12 minutes. After the reaction, 5 ml of 3M NaCl solution was added and 19 g of glycerin was further added to the reaction solution.

The resultant solution was adjusted to pH 8 with a 1M tris hydrochloride buffer of pH 7, left standing at 4°C for 12 hours, and then dialyzed with a 50 mM tris hydrochloride buffer/1M NaCl/pH 7.5 at 4°C for 12 hours. It was further dialyzed with 50 mM tris hydrochlorice buffer/0.1M NaCl/pH 7.5 at 4°C for 12 hours. The residual activity was inactivated until not more than 0.0001% by the addition of p-amidinophenylmethanesulfonyl fluoride.

The solution consequently obtained was added to 100 ml of benzamidine sepharose 6B column equilibrated with 50 mM tris hydrochloride buffer/0.1 M NaCl/pH 7.5 at 4°C, completely washed an unadsorbable component with said buffer, and eluted with 50 mM tris hydrochloride buffer/0.1 M benzamidine/0.1M NaCl/pH 7.5.

The eluate was dialyzed to expel benzamidine and obtain about 5 mg of an anhydridized coagulated factor X.

### Example 2

### 1) Production of anhydridized activated blood coagulation factor IX

To a solution of 8.5 mg of an activated blood coagulation factor IX derived from human blood plasma in 10 ml of 5 mM phosphate buffer/0.1M NaCl/pH 6.5, 30 µl of a 7% solution of phenyl methyl sulfonyl fluoride (PMSF) in methanol was added at intervals of 30 minutes until the total activity fell below 0.1%.

The produced solution was cooled to 0°C and the cooled solution and 0.5 ml of 1M NaOH added thereto were left reacting with each other for 12 minutes. After the reaction, 5 ml of 3M NaCl solution was added and 19 g of glycerin was further added to the reaction solution.

The resultant solution was adjusted to pH 8 with a 1M tris hydrochloride buffer of pH 7, left standing at 4°C for 12 hours, and then dialyzed with a 50 mM tris hydrochloride buffer/1M NaCl/pH 7.5 at 4°C for 12 hours. It was further dialyzed with 50 mM tris hydrochloride buffer/0.1M NaCl/pH 7.5 at 4°C for 12 hours. The residual activity was inactivated until not more than 0.0001% by the addition of p-amidinophenylmethanesulfonyl fluoride.

The solution consequently obtained was added to 100 ml of benzamidine sepharose 6B column equilibrated with 50 mM tris hydrochloride buffer/0.1 M NaCl/pH 7.5 at 4°C, completely washed an unadsorbable component with said buffer, and eluted with 50 mM tris hydrochloride buffer/0.1 M benzamidine/0.1M NaCl/pH 7.5.

The eluate was dialyzed to expel benzamidine and obtain about 4 mg of an anhydridized coagulated factor IX.

### Example 3

### 1) Production of anhydridized activated blood coagulation factor VII

To a solution of 3.1 mg of an activated blood coagulation factor VII derived from human blood plasma in 10 ml of 5 mM phosphate buffer/0.1M NaCl/pH 6.5, 30 µl of a 7% solution of phenyl methyl sulfonyl fluoride (PMSF) in methanol was added at intervals of 30 minutes until the total activity fell below 0.1%.

The produced solution was cooled to 0°C and the cooled solution and 0.5 ml of 1M NaOH added thereto were left reacting with each other for 12 minutes. After the reaction, 5 ml of 3M NaCl solution was added and 19 g of glycerin was further added to the reaction solution.

The resultant solution was adjusted to pH 8 with a 1M tris hydrochloride buffer of pH 7, left standing at 4°c for 12 hours, and then dialyzed with a 50 mM tris hydrochloride buffer/1M NaCl/pH 7.5 at 4°C for 12 hours. It was further dialyzed with 50 mM tris hydrochloride buffer/0.1M NaCl/pH 7.5 at 4°C for 12 hours. The residual activity was inactivated until not more than 0.0001% by the addition of p-amidinophenylmethanesulfonyl fluoride.

The solution consequently obtained was added to 30 ml of benzamidine sepharose 6B column equilibrated with 50 mM tris hydrochloride buffer/0.1 MNaCl/pH 7.5 at 4°C, completely washed an unadsorbable component with said buffer, and eluted with 50 mM tris hydrochloride buffer/0.1 M benzamidine/0.1M NaCl/pH 7.5.

The eluate was dialyzed to expel benzamidine and obtain about 1.7 mg of an anhydridized coagulated factor VII.

### Industrial Applicability

When the object of the first aspect of this invention, namely a substance capable of inhibiting a reaction between an activated blood coagulating factor and a substrate thereof by binding itself to said substrate in competition with said activated blood coagulating factor, is used as a thrombogenesis preventive, this substance is enabled to selectively inhibit exclusively the enzymatic activity which is aimed at.

Since the substance is capable of depressing the activity of an activated blood coagulation factor, it is not only highly useful as a preventive against thrombogenesis of novel mechanism, but also effectively usable in the control of enzymatic activity in other fields such as the process for purifying useful proteins.

When the object of the second aspect of this invention, namely the blood coagulation factor adsorbent, is used for the purpose of separating and purifying a blood coagulation factor, it is made possible to obtain a blood coagulation factor which has extremely low possibility of being contamination of fibrinogen and foreign proteins, etc.

## Claims

1. A substance capable of inhibiting a reaction between an activated blood coagulating factor and a substrate thereof by binding itself to said substrate in competition with said activated blood coagulating factor.

2. A substance according to claim 1, wherein said substance is formed from an amino acid sequence having one or more amino acids of an activated blood coagulation factor deleted therefrom, substituted therefor, or added thereto.

3. A substance according to claim 1 or claim 2, wherein said substance is formed by anhydridizing the active serine residue site of the activated blood coagulation factor possessing an active serine residue.

4. A substance according to claim 3, wherein said activated blood coagulation factor possessing an active serine residue is activated blood coagulation factor X.

5. A substance according to claim 3, wherein said activated blood coagulation factor possessing an active serine residue is activated blood coagulation factor IX.

6. A substance according to claim 3, wherein said activated blood coagulation factor possessing an active serine residue is activated blood coagulation factor VII.

7. A method for the production of a substance set forth in any of claims 1 - 6, **characterized by** performing the following steps
(1) a step of causing an active serine residue site of an activated blood coagulation factor possessing an active serine residue to react with a synthetic inhibitor,
(2) a step of performing an alkali treatment at a pH value in the range of 11.0 - 13.5, and
(3) a step of performing collection in the order mentioned above sequentially and allowing at least the step of performing collection to proceed with permitting coexistence of at least one compound selected from the group consisting of polyhydric alcohols and saccharides with a salt or an amphoteric electrolyte.

8. A blood coagulation factor adsorbent having a substance set forth in any of claims 1 - 6 fixed on a carrier.
